# EUROPEAN PATENT APPLICATION

(11) **EP 1 787 590 A1**
(43) Date of publication of application: **23.05.2007**
(21) Application number: 05780243.1
(22) Date of filing: 15.08.2005
(51) Int. Cl.: A61B 17/00

(54) **BLOOD VESSEL EXFOLIATION TOOL**

(30) Priority: 17.08.2004 JP 2004237264
(71) Applicant: JMS Co., Ltd., Hiroshima-shi, Hiroshima 730-8652 (JP)
(72) Inventor: SHIMIZU, Yasuhiro, Hiroshima-shi Hiroshima 7320064 (JP); HAYASHI, Shuro, JMS CO., LTD, Hiroshima-shi Hiroshima 7308652 (JP)
(74) Representative: Thoma, Michael
(86) International application number: PCT/JP2005/014900
(87) International publication number: WO 2006/019069

(57) **Abstract**

A blood vessel ablating tool **1** includes a metal-made tip end portion **20** in a slim bar-like shape and a resin-made base end portion **30** in a thick and long bar-like shape. A hook-like holding part **25** for hooking and holding a vein is formed at the tip end part of the tip end portion **20.** A curved portion **24** is formed at the base end of the holding part **25** of the tip end portion **20.** A tapered part **26** is formed at the base end of the curved part **24** of the tip end portion **20.** A tapered part **31** is formed in the base end portion **30** so as to continue to the tapered part **26** of the tip end portion **20.** The curved part **24** and the tapered parts **26, 31** are inserted sequentially between the vein hooked by the holding part **25** and supporting tissue to pull the vein in the direction away from the supporting tissue, thereby ablating the vein from the supporting tissue.

## Description

### Technical Field

The present invention relates to a blood vessel ablating tool for ablating a blood vessel from surrounding tissue.

### Background Art

Conventionally, as a method of treating a varicosis formed in a saphenous vein of blood vessels of human's lower limb or the like, there has been known a method of removing a part of the vein in which the varicosis is formed. In order to remove the varicosis, it is necessary to ablate the vein from the surrounding tissue, and it is general to ablate the vein by inserting a knife between a part of the vein which is exposed by dissecting a cuticle and the surrounding tissue. Ablation of the vein with the use of the knife, however, would cause serious damage to the surrounding tissue, and therefore, the vein is ablated by using, for example, a tool disclosed in Patent Document 1 for avoiding the damage by the knife.

The tool of Patent Document 1 is in a shape of a bar capable of being inserted in a vein. For ablating a vein with the use of this tool, the cuticle of a lower limb is dissected first to expose one end part of a to-be-ablated region of the vein, and then, the vein is cut at the exposed end part of the to-be-ablated region. The tool is inserted from the tip end thereof into the to-be-ablated region of the vein through the cut end part. When the tip end of the tool reaches the other end part of the to-be-ablated region of the vein, a part of the cuticle which corresponds to the tip end of the tool is dissected so that only the tip end of the tool is pushed out from the thus dissected cuticle. Subsequently, the one end part of the to-be-ablated region of the vein is joined to a step portion formed at the base end part of the tool by means of a thread, and then, the tip end of the tool is pulled out from the cuticle to allow the one end part of the vein to be pulled toward the other end part and to be brought inside the vein itself, so that the vein is ablated and removed from the surrounding tissue from the one end part to the other end part sequentially.
Patent Document 1: Japanese Patent Application Laid Open Publication No. 2003-61967A (Page 5, FIG. 1)

### Summary of the Invention

In the case where the vein joined to the bar-shaped tool is pulled as in Patent Document 1, however, it is necessary to fasten the vein to the tool firmly so that the vein does not slip off from the tool, of which operation is bothersome. Further, in vein ablation in Patent Document 1, the vein is brought inside the vein itself from the one end part, and therefore, the vein must be pulled strongly when the vein is brought inside the vein itself. For this reason, the vein would be broken in the middle of the to-be-ablated region, resulting in incomplete ablation of the vein.

The present invention has been made in view of the foregoing and has its object of enabling a treatment for ablating a blood vessel from surrounding tissue to be performed easily without breaking the blood vessel in the middle.

### Means for Solving the Problems

To attain the above object, in the present invention, a guide part for guiding a blood vessel in the direction away from the surrounding tissue is inserted between the blood vessel held by a holding part and the surrounding tissue.

Specifically, a blood vessel ablating tool of a first invention includes: a holding part formed at the tip end thereof for hooking and holding a blood vessel exposed from a dissected part of an organic cuticle; and a guide part which continues to the holding part and is to be inserted between the blood vessel held by the holding part and tissue surrounding the blood vessel, the guide part guiding, in course of the insertion thereof, the blood vessel in a direction away from the surrounding tissue.

With the above arrangement, the holding part hooks and holds the blood vessel exposed from the dissected part of the cuticle, thereby preventing the blood vessel from falling off from the blood vessel ablating tool. As the guide part is inserted between the blood vessel held by the holding part and the surrounding tissue, the blood vessel is pulled by the guide part in the direction away from the surrounding tissue to be ablated from the surrounding tissue. In ablation, the guide part pulls the blood vessel in the radial direction of the blood vessel, which means that the blood vessel is need not to be pulled strongly as in the case using the conventional tool in which the blood vessel is brought inside the blood vessel itself.

Referring to a second invention, in the blood vessel ablating tool of the first invention, the guide part includes a curved part in a bar-like shape which extends in an insertion direction and which is curved in a radial direction and a tapered part which continues from the base end in the insertion direction of the curved part and which extends in a direction opposite the insertion direction with a diameter thereof increasing.

With the above arrangement, though a slit between the blood vessel and the surrounding tissue is narrow at the beginning of ablation of the blood vessel from the surrounding tissue, the curved part, which is comparatively slim, is inserted into the narrow slit, enabling effortless insertion of the guide part between the blood vessel and the surrounding tissue. As the guide part is inserted between the blood vessel and the surrounding tissue, the blood vessel is guided by the curved part in the direction away from the surrounding tissue to be ablated from the surrounding tissue, thereby expanding the slit between the blood vessel and the surrounding tissue. Further insertion of the guide part leads to effortless insertion of the tapered part into the slit between the blood vessel and the surrounding tissue which has been expanded as described above. Accordingly, the blood vessel is further pulled in the direction away from the surrounding tissue by the tapered part, enabling ablation of the blood vessel over a wide range.

In a third invention, the blood vessel ablating tool of the second invention includes: a bar-shaped part which continues from the base end in the insertion direction of the tapered part of the guide part so as to extend in the direction opposite the insertion direction and which is to be inserted between the blood vessel and the surrounding tissue, wherein a tubular member, which is to be inserted between the blood vessel and the surrounding tissue together with the bar-like part, is detachably mounted around of the bar-shaped part, and the tubular member includes a tip end part in the insertion direction of which diameter decreases as it goes toward the tip end.

With the above arrangement, when the tubular member is detached from the bar-shaped part, the blood vessel of which length corresponds to the shape of the tapered part of the guide part is ablated from the surrounding tissue, as described above. On the other hand, when the tubular member is attached to the bar-shaped part, the blood vessel is pulled in the direction away from the surrounding tissue by the tip end in the insertion direction of the tubular member having an outer diameter lager than the tapered part, so that the blood vessel is ablated long enough when compared with the case where the tubular member is detached.

### Effects of the Invention

The first invention is provided with the holding part for holding the blood vessel exposed from the dissected part of the organic cuticle and the guide part for guiding the blood vessel in a direction away from the surrounding tissue in course of insertion thereof between the blood vessel held by the holding part and the surrounding tissue. Accordingly, the blood vessel is pulled in the radial direction of the blood vessel only by allowing the holding part to hold the blood vessel and inserting the guide part between the blood vessel and the surrounding tissue, thereby leading to easy ablation of the blood vessel from the surrounding tissue. It is not necessary for ablation to pull the blood vessel strongly by the guide part, thereby preventing the blood vessel from being broken in the middle.

In the second invention, the guide part includes the curved part and the tapered part of which diameter increases as it goes toward the base end in the insertion direction. Accordingly, the curved part can expand the slit between the blood vessel and the surrounding tissue effortlessly, and then, the blood vessel can be ablated from the surrounding tissue over a wide range by inserting the tapered part into the expanded slit. Hence, even in the case where the blood vessel to be ablated is long, the blood vessel can be ablated from the surrounding tissue easily with no breakage thereof involved.

The third invention is provided with the bar-shaped part extending continuously to the tapered part of the guide part, wherein the tubular member is detachably mounted around the bar-shaped part. Accordingly, attachment and detachment of the tubular member results in easy ablation of blood vessels different in length with the holding part and the guide part unchanged.

### Brief Description of the Drawings

[FIG. **1**] FIG. **1** is a side view of a blood vessel ablating tool according to Embodiment 1.
[FIG. 2] FIG. **2(a)** is a side view of a tip end portion, and FIG. **2(b)** is a side view of a base end portion.

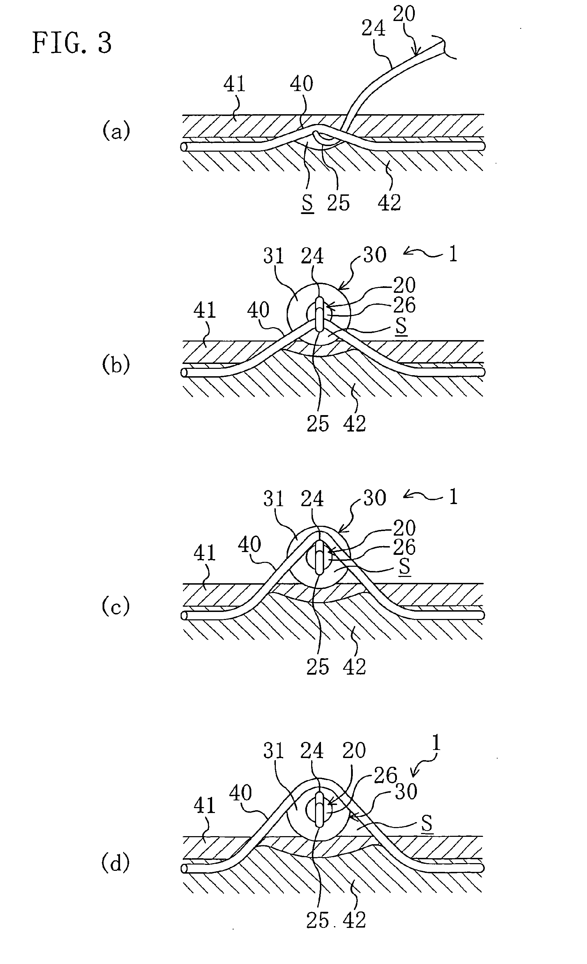

[FIG.**3**] FIG. **3** explains a procedure for ablating a saphenous vein from supporting tissue with the use of the blood vessel ablating tool, wherein FIG. **3(a)** is an illustration showing a state that a holding part hooks the vein, FIG. **3(b)** is an illustration as viewed from the holding part side and shows a state that the blood vessel ablating tool is brought down, FIG. **3(c)** is an illustration showing a state that a curved part is inserted between the vein and the supporting tissue, and FIG. **3(d)** is an illustration showing a state that a tapered part is inserted between the vein and the supporting tissue.
[FIG. **4]** FIG. **4** is a partial sectional view of a blood vessel ablating tool according to Embodiment 2.
[FIG. **5]** FIG. **5** is a view corresponding to FIG. **1** according to Modified Example 1 of Embodiments 1 and 2.
[FIG. **6]** FIG. **6** is a view corresponding to FIG. **2(b)** according to Modified Example 1 of Embodiments 1 and 2.
[FIG. 7] FIG. 7 is a view corresponding to FIG. **1** according to Modified Example 2 of Embodiments 1 and 2.

### Explanation of Reference Numerals

- **1**: blood vessel ablating tool
- **24**: curved part (guide part)
- **25**: holding part
- **26**: tapered part (guide part)
- **30**: base end portion (bar-shaped part)
- **40**: vein (blood vessel)
- **42**: supporting tissue (surrounding tissue)
- **50**: first tubular member
- **52**: second tubular member

### Best Mode for Carrying out the Invention

Embodiments of the present invention will be described below with reference to the accompanying drawings. It should be noted that the following preferred embodiments describe only substantial examples and are not intended to limit any of the present invention, applicable articles thereof, and the usage thereof.

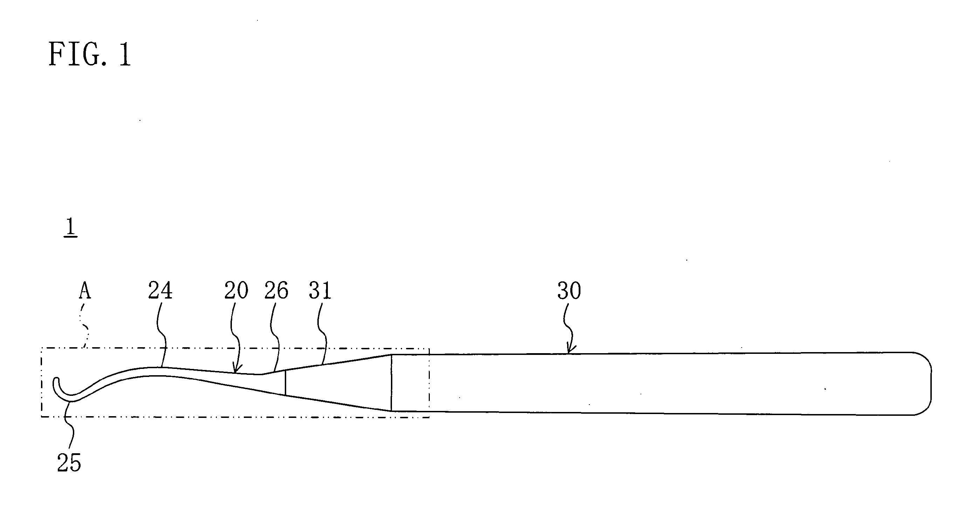

<Embodiment 1 of the Invention>
FIG. **1** shows a blood vessel ablating tool **1** according to an embodiment of the present invention, which is a tool used for ablating, for removing a part of a vein **40** in which a varicosis is formed, the vein **40** (shown in FIG. 3) from surrounding tissue **42.**

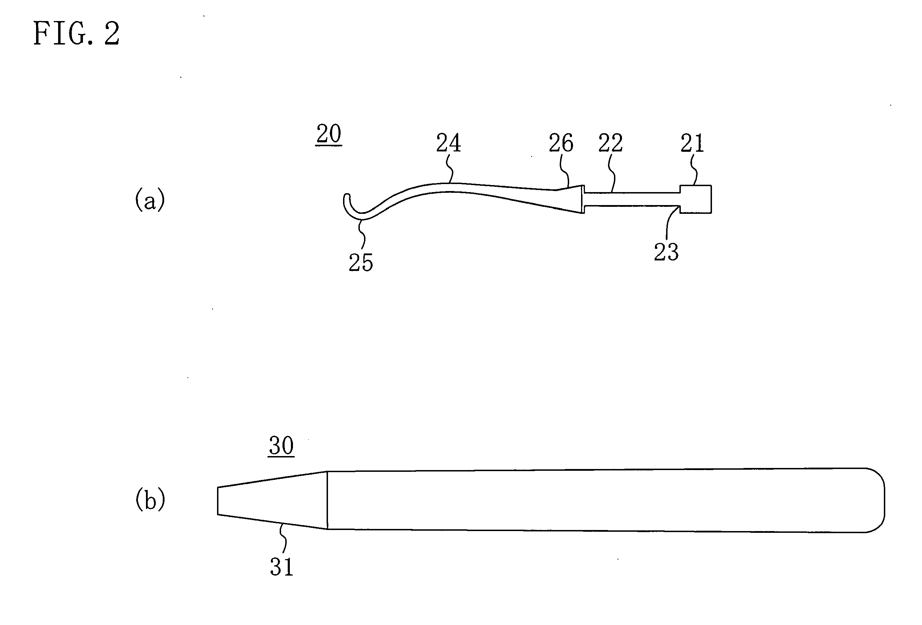

The blood vessel ablating tool 1 includes a metal-made tip end portion **20** in a slim bar-like shape and a resin-made base end portion **30** in a thick bar-like shape which is arranged at the base end (the right side in FIG. **1** and FIG. **2)** of the tip end portion **20.**

The tip end portion **20** has a dimension of approximately 60 mm in the longitudinal direction, and a base end part thereof, which is approximately one third of the tip end portion 20 from the base end thereof, extends straight substantially. The tip end portion **20** has a large diameter part **21** at the base end thereof and a small diameter part **22** at the tip end of the large diameter part **20** (the left side in FIG. **1** and FIG. **2)** so that the large diameter part **21** and the small diameter part **22** form a step part **23.** A part of the tip end portion **20** on the tip end side of the small diameter part **22** has a diameter larger than the small diameter part **22** and continues to a tapered part **26** of which diameter decreases as it goes toward the tip end. The tapered part **26** continues at the tip end thereof to a curved part **24** curved towards one side in the radial direction of the tip end portion **20.** In other words, the tapered part **26** increases its diameter as it goes toward the base end thereof while continuing to the base end of the curved part **24.** The curved part **24** and the tapered pat **26** compose a guide part of the present invention.

At the tip end part of the tip end portion **20,** a hook-like holding part **25** for hooking and holding the vein **40** is formed continuously. The holding part **25** is formed in such a fashion that the base end part thereof is comparatively steeply curved in a radial direction of the tip end portion **20** opposite to the curved part **24** continuously from the curve part **24** toward the tip end thereof and the tip end part thereof is curved in the opposite direction to the base end part thereof so as to extend up to the central part in the radial direction of the tip end portion **20.** The tip end of the holding part **25** is open so that the vein **40** is introduced into the holding part **25** from the opening.

The tip end portion **20** is so formed that the center line of the base end part thereof aligns with the center line of the base end portion **30,** and the base end part of the tip end portion **20** is inserted in the tip end part of the base end portion **30** so as to be assembled with the base end portion **30** when forming the base end portion **30.** In this assembled state, the step part **23** of the tip end portion **20** inhibits the tip end portion **20** from falling off from the base end portion **30.**

On the other hand, the base end portion **30** has a dimension of approximately 80 mm in the longitudinal direction and has an outer diameter at the tip end thereof substantially the same as the outer diameter of a part of the tip end portion **20** which is around the boundary with the base end portion **30** so that the outer face of the base end portion **30** continues to the outer face of the tip end portion **20.** A tapered part **31** is formed at a part of the base end portion **30** which is located on the base end side of the boundary between the base end portion **30** and the tip end portion **20** so as to increase its diameter as it goes toward the base end of the base end portion **30.** A part of the base end portion **30** which is located on the base end side of the tapered part **31** has entirely a circular section and extends straight substantially. Reference letter A in FIG. **1** denotes a detachable cap for covering the tip end part of the blood vessel ablating tool **1** in nonuse.

Description will be given below with reference to FIG. **3** to a procedure for removing a part of the saphenous vein **40** in which a varicosis is formed with the use of the thus structured blood vessel ablating tool **1.** In FIG. **3,** reference numeral **41** denotes a cuticle and hypodermal tissue lying under the cuticle, such as fats and the like, and supporting tissue **42** for supporting the surroundings of the saphenous vein **40** lies under the cuticle and the hypodermal tissue **41.**

First, the cuticle and the hypodermal tissue **41** which correspond to a part in which the varicosis is formed is dissected, and further, a part of the supporting tissue **42** which is upper than the vein **40** is dissected so that the part of the vein **40** in which the varicosis is formed is exposed. The exposed part of the vein **40** is a to-be-ablated region. Though not shown, a clamp or the like clips and raises so as to ablate the central part of the to-be-ablated region from the underlying supporting tissue **42** for creating a slit S between the ablated part and the supporting tissue **42.** As shown in FIG. **3(a),** the operator inserts the tip end of the holding part **25** into the space S and grasps and moves the base end portion **30** so that the holding part **25** scoops up, hooks, and holds the vein **40.** Subsequently, as shown in FIG. **3(b),** the blood vessel ablating tool 1 is brought down in the direction that the center line of the base end portion **30** is intersected with the direction that the vein **40** extends so that the outer face of the base end portion **30** is in contact with the cuticle. When doing so, the vein **40** is pulled upward by the holding part **25,** namely, is pulled in the radial direction of the vein **40** to be ablated from the supporting tissue **42,** thereby expanding the slit S. In the state that the vein **40** is held by the holding part 25, which has a hook-like shape, the vein **40** is prevented from falling off from the holding part **25** even if the blood vessel ablating tool 1 is brought down as above.

Thereafter, the tip end portion **20** of the blood vessel ablating tool **1** is inserted from the tip end thereof between the vein **40** and the supporting tissue **42.** In insertion, though the slit S between the vein **40** and the supporting tissue **42** is comparatively narrow, the tip end portion **20,** which is comparatively slim, can be inserted easily and effortlessly. As the tip end portion **20** is inserted, the vein **40** slides on the outer face of the tip end portion **20,** reaches the curved part **24,** and then, is pulled upward by the curved part **42,** as shown in FIG. 3(c), to be ablated further from the supporting tissue **42.**

When the blood vessel ablating tool **1** is moved further in the insertion direction, the vein **40** slides on the tapered part **26** and is pulled upward to be ablated further from the supporting tissue **42,** as shown in FIG. **3(d),** and further, the vein **40** slides on the tapered part **31** of the base end portion **30** to be ablated further from the supporting tissue **42.** Then, the tip end portion **20** of the blood vessel ablating tool **1** is pulled so as to pull out the base end portion **30** from the slit S between the vein **40** and the supporting tissue **42.** For removing the ablated vein **40,** the operator clips up the vein with the use of a clamp or the like and cuts a part near each end of the to-be-ablated region.

Thus, in the blood vessel ablating tool **1** according to the present embodiment, only by allowing the holding part **25** to hook and hold the vein **40** and inserting the tip end portion **20** and the base end portion **30** between the vein **40** and the supporting tissue **42,** the vein **40** is pulled in the direction away from the supporting tissue **42** by the curved part **24** and the tapered parts **26, 31,** thereby ablating the vein **40** from the supporting tissue **42** easily. In ablation, the direction that the vein **40** is pulled is the radial direction of the vein **40,** which means that strong force for pulling the vein **40,** which the conventional tool has required, is unnecessary, preventing the vein **40** from being broken in the middle.

Further, since the holding part **25** for hooking the vein **40** is formed integrally with the curved part **24** and the tapered parts **24, 36** for ablating the vein **40,** as described above, the vein **40** can be ablated with the use of only the blood vessel ablating tool **1,** eliminating the need of preparing additional tools, such as a Kocher clamp and the like.

Moreover, the curved part **24** in a slim bar-like shape is formed at the tip end part in the insertion direction, and the tapered part **26** of which diameter increases as it goes toward the base end thereof continues to the base end in the insertion direction of the curved part **24.** Accordingly, the curved part **24** can expand the slit S between the vein **40** and the supporting tissue **42** effortlessly even if the slit S is narrow at the beginning of ablation of the vein **40,** and the tapered part **26** of the tip end portion **20** can be inserted in the thus expanded slit S smoothly, As a result, the vein **40** can be ablated from the supporting tissue **42** over a wide range. In addition, after the vein **40** is ablated by the tapered part **26,** the tapered part **31** of the base end portion **30,** which has a comparatively large diameter, is further inserted. This facilitates ablation of the vein **40** over a further wide range. Hence, the vein **40,** which would be to be ablated long, can be ablated easily with no breakage thereof involved.

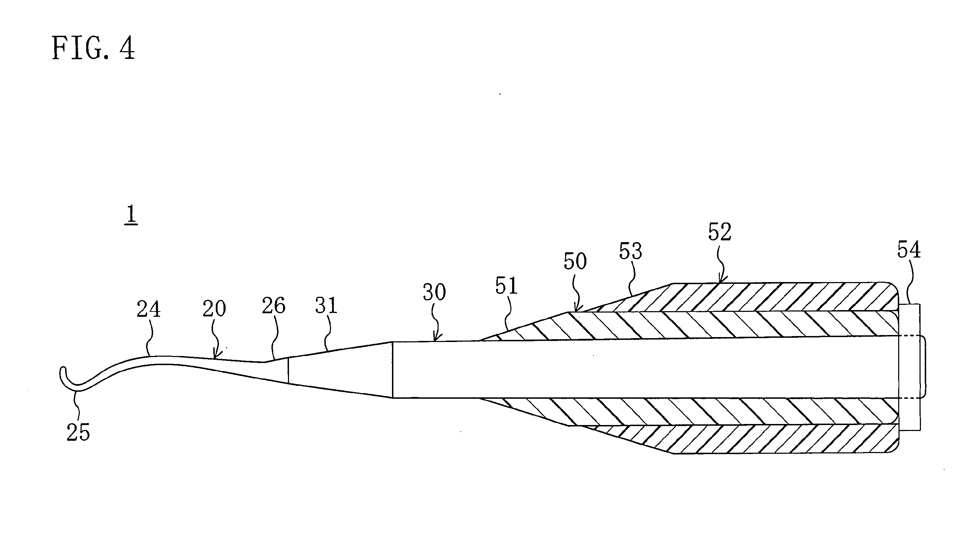

<Embodiment 2 of the Invention>
FIG. **4** shows a blood vessel ablating tool **1** according to Embodiment 2 of the present invention. The blood vessel ablating tool **1** is different from that according to Embodiment 1 in only that resin-made first and second tubular members **50, 52** are provided around the base end portion **30** as a bar-shaped part, and the other parts thereof are the same as those in Embodiment **1.** Therefore, the same reference numerals are assigned to the same parts as those in Embodiment 1 for omitting the description thereof, and only the difference will be described in detail.

The first tubular member **50** is mounted detachably around the base end portion **30,** wherein the dimension in the direction of the center line thereof is set shorter than that of the base end portion **30** in the direction of the center line. The first tubular member **50** includes at the tip end part thereof (the left side in FIG. **4)** a tapered part **51** of which diameter increases as it goes toward the base end. The first tubular member **50** is so formed that the inner peripheral face thereof adheres to the outer peripheral face of the base end portion **30.**

The second tubular member **52** is mounted detachably around the first tubular member **50,** wherein the dimension in the direction of the center line thereof is set shorter than that of the first tubular member **50** in the direction of the center line. The second tubular member **52** includes at the tip end part thereof a tapered part **53** similar to the tapered part **51** of the first tubular member **50.** The second tubular member **52** is so formed that the inner peripheral face thereof adheres to the outer peripheral face of the first tubular member **50.** The first tubular member **50** and the second tubular member **52** are formed separately, so that the first tubular member **50** only can be mounted to the base end portion **30.**

At the base end of the base end portion **30,** a stopper **54** is provided for preventing the tubular members **50, 52** from falling off from the base end portion **30.** The stopper **54** is in an annular shape having an outer diameter larger than the inner diameter of the second tubular member **52.** A female screw part (not shown) is formed in the inner peripheral face of the stopper member **54** while a male screw part (not shown) is formed at the base end part in the outer peripheral face of the base end portion **30** so that the female screw part of the stopper **54** is engaged with the male screw part of the base end portion **30** to fix the stopper **54** to the base end portion **30,** thereby preventing the tubular members **50, 52** from falling off from the base end of the base end portion **30.**

When the first tubular member **50** is mounted to the base end portion **30,** the vein **40** under which the respective tapered parts **26, 31** of the tip end portion **20** and the base end portion **30** have passed is pulled further upward when the tapered part **51** of the first tubular member **50** passes thereunder. This ensures the ablated length of the vein **40** long. As well, when the second tubular member **52** is mounted, the vein **40** under which the tapered part **51** of the first tubular member **50** has passed is pulled further upward when the tapered part **53** of the second tubular member **52** passes thereunder, ensuring the ablated length of the vein **40** further long.

Hence, in the blood vessel ablating tool **1** according to the present embodiment, attachment or detachment of the first tubular member **50** only or together with the second tubular member **52** facilitates ablation of veins **40** different in length according to presentation with the tip end portion **20** and the base end portion **30** unchanged.

It is noted that though the tip end portion and the base end portion in the insertion direction of the blood vessel ablating tool **1** in the above embodiments are made of metal and resin, respectively, they may be made of the same material. When they are made of the same material, the base end portion and the tip end portion in the insertion direction may be formed integrally.

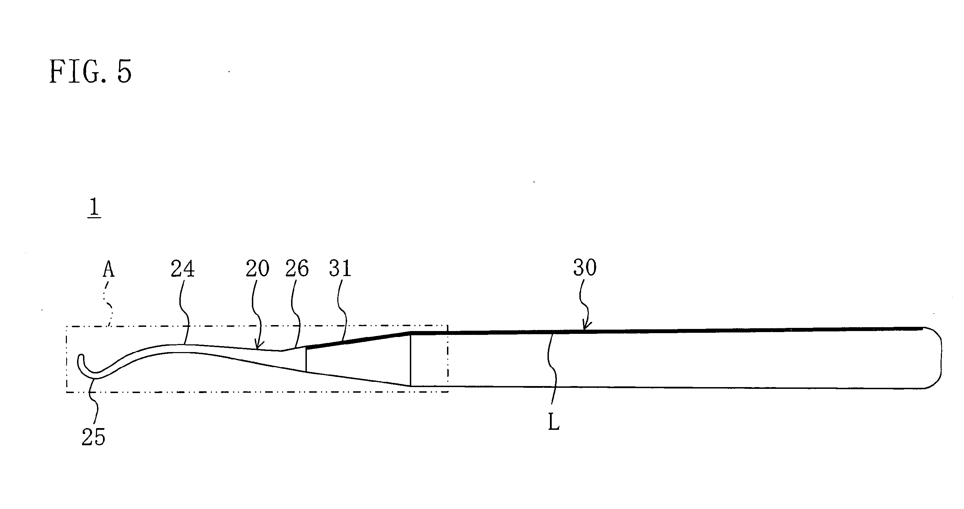

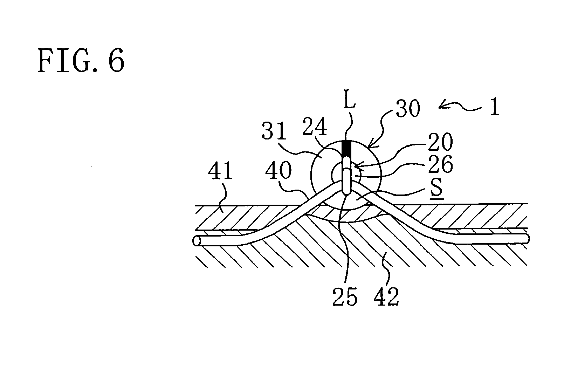

Further, referring to Modified Example 1 of Embodiments 1 and 2, a mark **L** may be provided in the base end portion **30,** as shown in FIG. **5** and FIG. **6.** The mark **L** serves to indicate which direction the opening of the holding part **25** faces when the holding part **25** is inserted in the organic tissue and cannot be seen from the operator. Specifically, as shown in FIG. **6,** the mark **L** is provided at a part of the outer peripheral face of the base end portion **30** which corresponds substantially to the opening of the holding part **25** when viewing the blood vessel ablating tool **1** from the holding part **25** side, and extends continuously from one end over to the other end in the longitudinal direction of the base end portion **30.** The mark **L** is provided in such a manner that a part in a line shape of the outer peripheral face of the base end portion **30** is colored in a color different from the color of the member composing the base end portion **30.**

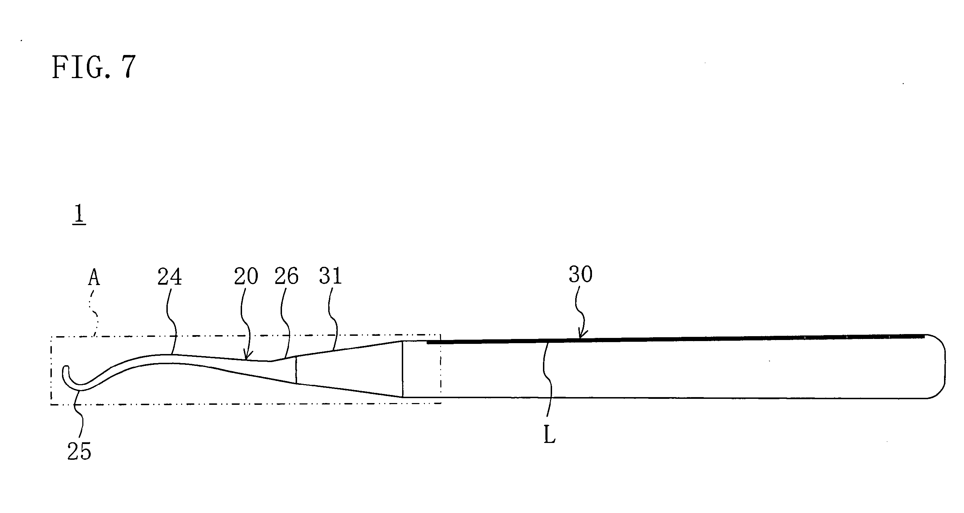

Alternatively, as Modified Example **2** shown in FIG. **7,** the mark **L** may be provided in a part other than the tapered part **31.** The base end portion **30** of Modified Example 2,which is generally called a two-tone molded article, is formed in such a manner that a resin material for forming the base end portion **30** is injected into a molding die and is hardened, and then, a resin material for forming the mark **L** is injected into the molding die without removing the die and is hardened. When the mark **L** is integrally formed in the base end portion **30** in this way, the outer peripheral face of the base end portion **30** becomes smooth with no projection formed. This enables the vein **40** to be ablated smoothly without being hooked at the base end portion **30.**

The aspect of the mark **L** is not limited to the above only if the operator can recognize the mark **L** visually or at a touch. For example, though not shown, a protrusion or a recess may be formed as the mark **L** in the outer peripheral face of the base end portion **30.**

Optionally, the blood vessel ablating tool **1** may be used for ablating an artery other than the vein **40.**

### Industrial Applicability

As described above, the blood vessel ablating tool according to the present invention can be used for treating, for example, a varicosis formed in a saphenous vein of a lower limb.

## Claims

1. A blood vessel ablating tool comprising:
a holding part formed at the tip end thereof for hooking and holding a blood vessel exposed from a dissected part of an organic cuticle; and
a guide part which continues to the holding part and is to be inserted between the blood vessel held by the holding part and tissue surrounding the blood vessel, the guide part guiding, in course of the insertion thereof, the blood vessel in a direction away from the surrounding tissue.

2. The blood vessel ablating tool of Claim 1,
wherein the guide part includes a curved part in a bar-like shape which extends in an insertion direction and which is curved in a radial direction and a tapered part which continues from the base end in the insertion direction of the curved part and which extends in a direction opposite the insertion direction with a diameter thereof increasing.

3. The blood vessel ablating tool of Claim 2, comprising:
a bar-shaped part which continues from the base end in the insertion direction of the tapered part of the guide part so as to extend in the direction opposite the insertion direction and which is to be inserted between the blood vessel and the surrounding tissue,
wherein a tubular member, which is to be inserted between the blood vessel and the surrounding tissue together with the bar-like part, is detachably mounted around of the bar-shaped part, and
the tubular member includes a tip end part in the insertion direction of which diameter decreases as it goes toward the tip end.
